# EUROPÄISCHE PATENTSCHRIFT

(11) **EP 1 372 749 B1**
(45) Veröffentlichungstag und Bekanntmachung des Hinweises auf die Patenterteilung: **06.10.2004**
(21) Anmeldenummer: 01916831.9
(22) Anmeldetag: 02.04.2001
(51) Int. Cl.: A61L 27/32

(54) **BIOAKTIVE OBERFLÄCHENSCHICHT, INSBESONDERE FÜR MEDIZINISCHE IMPLANTATE UND PROTHESEN**
BIOACTIVE SURFACE LAYER, PARTICULARLY FOR MEDICAL IMPLANTS AND PROSTHESES
COUCHE SUPERFICIELLE BIOACTIVE, NOTAMMENT POUR PROTHESES ET IMPLANTS MEDICAUX

(43) Veröffentlichungstag der Anmeldung: 02.01.2004
(73) Patentinhaber: Stratec Medical AG, 4436 Oberdorf (CH); Dr.h.c. Robert Mathys Stiftung, 2544 Bettlach (CH)
(72) Erfinder: FRAUCHIGER, Vinzenz, M., CH-4514 Lommiswil (CH); SCHLOTTIG, Falko, CH-4414 Fülinsdorf (CH); TEXTOR, Marcus, CH-8200 Schaffhausen (CH); GASSER, Beat, CH-3063 Ittigen (CH)
(74) Vertreter: Lusuardi, Werther Giovanni, Dr.
(86) Internationale Anmeldenummer: PCT/CH2001/000210
(87) Internationale Veröffentlichungsnummer: WO 2002/078759

(56) Entgegenhaltungen:
- EP-A- 0 806 212
- WO-A-98/13539
- US-A- 4 801 300
- US-A- 5 711 763
- US-A- 6 013 591
- US-A- 6 129 928

## Beschreibung

Die Erfindung bezieht sich auf eine bioaktive Oberflächenschicht gemäss dem Oberbegriff des Patentanspruchs 1 sowie auf ein Verfahren zur Herstellung einer calciumphosphathaltigen, bioaktiven, porösen Oberflächenschicht gemäss dem Oberbegriff des Anspruchs 16.

Aus der US-A-5 478 237 ISHIZAWA ist ein Zahnimplantat aus Titan bekannt, welches mittels einer anodischen Oxidation des Substrates mit einer Oberflächenschicht versehen ist, welche Calcium- und Phosphat-lonen enthält und zwar in Form von Hydroxylapatit-Kristallen. Nachteilig bei dieser Beschichtung ist der Umstand, dass die Hydroxylapatit-Kristalle im wesentlichen unlöslich sind, so dass die Calcium- und Phosphat-lonen nicht in den Knochen eingebaut werden können. Ein weiterer Nachteil dieser bekannten, kristallinen Beschichtung liegt in ihrer Sprödigkeit, welche die Gefahr eines Abbtätterns der Beschichtung in sich birgt. Schliesslich handelt es sich beim offenbarten Herstellungsverfahren um einen Zweistufenprozess, was als nachteilig zu betrachten ist.

Hier will die Erfindung Abhilfe schaffen. Der Erfindung liegt die Aufgabe zugrunde eine bioaktive, poröse, Calciumphosphat (CaP) enthaltende Oberflächenschicht zu schaffen, die Calciumphosphate einer hohen Löslichkeit aufweist, so dass sie als Spenderin von Calciumphosphat für die Knochenbildung wirken kann.

Die Erfindung löst die gestellte Aufgabe mit einer Oberflächenschicht, welche die Merkmale des Anspruchs 1 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Oberflächenschicht mit in der Porenstruktur löslich eingelagerten Calciumphosphatphasen der Knochen schneller in die poröse Trägerstruktur einwachsen kann. Dabei handelt es sich nicht nur um eine oberflächliche Imprägnierung mit Calcium- und Phosphat-lonen, sondern auch um eine durch das erfindungsgemässe Verfahren in Abhängigkeit der Wahl der Prozessparameter erzielbare, der bestehenden Oberflächenstruktur überlagerte Mikrostrukturierung mit einem Einbau amorpher oder nanokristalliner, und damit löslicher Calciumphosphat-Phasen in die durch anodische Oxidation unter Funkenentladung gebildete Metalloxid-Schicht.
Das erfindungsgemässe Verfahren hat den Vorteil, dass es ein Einstufenprozess ist, der schnell abläuft und sich auch für komplexe geometrische Formen eignet.

Bei einer bevorzugten Ausführungsform der Erfindung enthält die Oberflächenschicht zusätzlich Hydroxylapatit.

Vorzugsweise liegt das Verhältnis Ca/P über die gesamte Oberflächenschicht im Bereich von 1,0 bis 1,8, d.h. etwa ähnlich wie für TCP, HA und Ca-defizienten HA. Es hat sich gezeigt, dass ein Ca/P Verhältnis, welches in der Nähe des natürlichen, anorganischen Knochenmaterials Hydroxylapatit liegt (1,67), ein optimales Ca/P Verhältnis bezüglich der in Lösung gehenden Schichtanteile darstellt.

Bei einer weiteren bevorzugten Ausführungsform der Erfindung machen die amorphen oder nanokristallinen Calciumphosphate und/oder der Hydroxylapatit 1 bis 40 Volumen % der gesamten Oberflächenschicht aus. Da die Calciumphosphat-Phase in die aus dem Substratmaterial herausgewachsenen Schicht integriert ist, ist eine bessere Adhäsion gegeben.

Bei einer weiteren bevorzugten Ausführungsform der Erfindung enthält die Oberflächenschicht einen Anteil von 25 bis 95 Atomprozenten Metalloxid, vorzugsweise von 30 bis 80 Atomprozenten. Es hat sich gezeigt, dass bei diesen Metalloxidanteilen die mechanische und chemische Beständigkeit der Schichtmatrix optimal ist.

Bei einer weiteren bevorzugten Ausführungsform der Erfindung liegt das Metalloxid in Form von Kristallen vor, vorzugsweise mit einer Grösse der Kristalle von 10 bis 150 Nanometer, was für die Löslichkeit der Ca- und P-lonen günstig ist.

Bei einer weiteren bevorzugten Ausführungsform der Erfindung ist das Metalloxid Titandioxid, vorzugsweise in der Form von Anatas oder Rutil, welche eine spezifische Kristallstruktur aufweisen.
Bei einer weiteren bevorzugten Ausführungsform der Erfindung sind die in der Oberflächenschicht eingelagerten Ca²⁺-lonen und PO₄³⁻-lonen über die ganze Metalloxid-Schicht verteilt. Eine solche homogene Oberflächenzusammensetzung hat im Gegensatz zu einer rein oberflächlichen Imprägnierung den Vorteil, dass sie die Freisetzung genügend grosser Mengen an Calcium und Phosphat pro Flächeneinheit für die Knochenbildung ermöglicht.
Bei einer weiteren bevorzugten Ausführungsform der Erfindung beträgt die Dicke der Oberflächenschicht 0,5 bis 10,0 µm. Eine solche, relativ dünne Schicht ist vorteilhaft bezüglich Adhäsion, speziell bei Scherbeanspruchung. Sie garantiert im weiteren einen optimalen Schutz vor Korrosion und insbesondere vor mechanischem Abrieb, ohne jedoch die Gefahr eines spröden Verhaltens und/oder von Spannungen bei mechanischer Beanspruchung (im speziellen bei Scherbeanspruchung) oder thermischer Belastung aufkommen zu lassen, wie dies häufig bei zu dicken Schichten der Fall ist.

Bei einer weiteren bevorzugten Ausführungsform der Erfindung enthalten die Poren der porösen Oberflächenschicht pharmakologisch wirksame Substanzen, vorzugsweise Peptide, Wachstumsfaktoren, Bone Morphogenetic Proteins, Antibiotika oder Entzündungshemmer. Der Vorteil dieser zusätzlichen Substanzen liegt in ihrer induktiven Wirkung. Solche Wirkstoffe ermöglichen die biologische oder biochemische Unterstützung des Heilungsprozesses des Knochengewebes. Die Freisetzungskinetik (zeitliche Abgabe des Wirkstoffes oder der Wirkstoffe pro Flächeneinheit) kann dabei über die Wahl der Porengrösse (Durchmesser) und des Porenvolumens (Porendurchmesser, Porendichte) gesteuert werden.

Die erfindungsgemässe Oberflächenschicht wird vorzugsweise auf ein Substrat aufgebracht, welches eines oder mehrere der Elemente Ti, Zr, Ta, Nb, Al, V, Mg oder deren Legierungen enthält. Die Elemente Ti, Zr, Ta, Nb, Al, V, Mg werden auch als Ventilmetalle bezeichnet (siehe M.M.Lohrengel,"Thin anodic oxide layers on aluminium and other valve metals", Materials Science and Engineering, R11, No. 6, December 15, 1993). Die Beschichtung solcher Substrate mit der erfindungsgemässen Oberflächenschicht hat sich als besonders vorteilhaft erwiesen.

Die auf diesen Substraten aufgebrachte, erfindungsgemässe Oberflächenschicht besteht vorzugsweise mindestens teilweise aus nanokristallinen oder mikrokristallinen Oxiden oder Mischoxiden des metallischen Substrats. Es resultiert daraus eine mindestens osteokonduktive, strukturierte, poröse Schicht und damit ein direkter Knochenkontakt.

Der Hauptbestandteil der Oberflächenschicht besteht vorzugsweise aus dem nanooder mikrokristallinen Oxid oder Mischoxid des metallischen Grundmaterials, typischerweise mit einem Anteil von 60 bis 99 Volumen %.

Bei einer bevorzugten Ausführungsform besteht das Substrat aus Kunststoffen, vorzugsweise aus Polyoxymethylen (POM), Polyetheretherketon (PEEK), Polyaryletherketon (PAEK), Polyetherimid (PEI) oder flüssigkristallinem Polymer (LCP), Polymethylpenten (PMP), Polysulfon (PSU), Polyäthersulfon (PESU oder PES), Polyäthylenterephthalat (PETP), Polymethylmethacrylat (PMMA) oder ultrahochmolekularem Polyäthylen (UHMW-PE), wobei das Substrat mit einer metallischen Schicht aus Ventilmetallen versehen ist. Damit ist eine Anwendung auf elastischen Implantaten und Prothesen möglich.

Das erfindungsgemässe Verfahren zur Herstellung einer calciumphosphathaltigen, bioaktiven, porösen Oberflächenschicht auf Ventilmetallen oder deren Legierungen wie auch Ventilmetallbeschichtungen auf einem Substrat wird durch die Merkmale des Anspruchs 16 charakterisiert.

Als Chelatbildner eigenen sich die folgenden Substanzen:
a) anorganische Carbonsäuren, insbesondere Bi- oder Multidentate, bzw. deren Carboxylate, im speziellen: Zitronensäure, Weinsäure, Nitriloessigsäure (NTA), Ethylendiamin-tetraessigsäure (EDTA), 1,2-Cyclohexandiamin-tetraessigsäure (CDTA), Diethylen-triaminessigsäure (DTPA), 2-Hydroxyethyl-ethylen-diamin-triessigsäure, Triethylentetraminhexaessigsäure (TTHA);
b) Ketone, insbesondere Di- oder Multiketone, im speziellen β-Diketon (CH₃-CO-CH₂-CO-CH₃);
c) Organophosphorsäuren, bzw. -phosphate (mit ≥ 2 Phosphatgruppen);
d) Organophosphonsäuren, bzw. -phosphonate (mit ≥ 2 Phosphonatgruppen);
e) Organophosphorige Säuren, bzw. -phosphite (mit ≥ 2 Phosphitgruppen); sowie geeignete Salze aller vorstehenden Substanzen.

Als anorganischer Komplexbildner eignet sich im speziellen CaX₆⁴⁻, insbesondere mit X = Fluorid.
Die Konzentration des Komplexbildners beträgt vorteilhafterweise 0,06 bis 0,24 mol/l. Vorteilhafterweise wird als Phosphatverbindung das Calcium-Bis-(Dihydrogenphosphat) verwendet, typischerweise mit einer Konzentration von 0,01 bis 0,05 mol/l. Die wasserlöslichen Calciumverbindungen (vorzugsweise Calciumacetat) werden vorzugsweise mit einer Konzentration von 0,03 bis 0,15 mol/l verwendet.

Als basischer Zusatz eignen sich Hydroxidverbindungen , vorzugsweise Natrium- oder Kaliumhydroxid, typischerweise mit einer Konzentration von 0,5 bis 1,5 mol/l.

Bei einer bevorzugten Ausführungsform des Beschichtungsverfahrens werden die Parameter des Anodisierungsprozesses (Spannung, Strom, Frequenz, Beschichtungsdauer, Badgeometrie etc.) so gewählt, dass sich die Schicht durch Reaktion zwischen dem Substrat und dem Elektrolyten bildet, wobei die bereits gebildete Schicht durch die Funkenentladung partiell rekristallisiert. Die Temperatur des Elektrolyten beträgt während des Beschichtungungsvorganges zweckmässigerweise 10 bis 90 °C, vorzugsweise 20 bis 75 °C.
Vorzugsweise werden Substrate aus den Elementen Ti, Zr, Ta, Nb, Al, V, Mg oder deren Legierungen oder auch sperrschichtbildende Metallbeschichtungen auf beliebigen Substraten beliebiger Form und Oberflächenbeschaffenheit allseitig oder partiell beschichtet. Vorteilhafterweise erfolgt eine Beeinflussung der Oberflächentopographie oder -morphologie durch chemische und/oder mechanische Vorbehandlungen der Ausgangsoberfläche, wobei die chemische Vorbehandlung ein Ätzverfahren und die mechanische Vorbehandlung ein Strahlprozess sein kann.

Die Erfindung und Weiterbildungen der Erfindung werden im folgenden anhand eines Ausführungsbeispiels noch näher erläutert.

### Beispiel :

Eine zylindrische Scheibe aus Reintitan (Höhe 1 mm, Durchmesser 7 mm) wurde bei einer Temperatur von 25°C in folgender Elektrolytlösung:
10,5 g Calcium-dihydrogenphosphat
22 g Calcium-diacetat
74 g Ethylen-diamin-tetraessigsäure-dinatrium-salz
13 g Natriumhydroxid
mit Reinstwasser auf 1 Liter Gesamtvolumen eingestellt;
bei einer Stromstärke von 80 mA während 90 Sekunden galvanostatisch beschichtet.
Die erhaltene, hellgraue Beschichtung wies folgende Konzentrationsverhältnisse in Atomprozenten auf: Ca/Ti: 1,05 P/Ti: 0,83 und Ca/P: 1,27.

## Patentansprüche

1. Bioaktive Oberflächenschicht, insbesondere für medizinische Implantate und Prothesen, wobei
A) ein variabler Anteil der Oberflächenschicht aus Calciumphosphatphasen besteht,
B) die Oberflächenschicht einen Anteil von 25 bis 95 Atomprozenten Metalloxid des metallischen Grundmaterials enthält;
C) die Dicke der Schicht zwischen 0,1 und 50,0 µm beträgt,
D) die Oberflächenschicht porös ausgebildet ist,
E) die Oberflächenschicht amorphe oder nanokristalline Calciumphösphate enthält; und
F) das Verhältnis Ca/P über die gesamte Öberflächenschicht im Bereich von 0,5 bis 2,0 liegt;
**dadurch gekennzeichnet, dass**,
G) die in der Oberflächenschicht eingelagerten Ca-lonen und PO₄-lonen über die ganze Metalloxid-Schicht verteilt sind;
H) die Porendichte an der Oberfläche der Oberflächenschicht zwischen 10⁴ bis 10⁸ Poren/mm² liegt; und
I) die amorphen oder nanokristallinen Calciumphosphate sowie allfällige Hydroxylapatit-Anteile 1 bis 40 Volumen % der gesamten Öberflächenschicht ausmachen.

2. Oberflächenschicht nach Anspruch 1, **dadurch gekennzeichnet, dass** die Oberflächenschicht aus Hydroxylapatit besteht.

3. Oberflächenschicht nach Anspruch 1, **dadurch gekennzeichnet, dass** die Oberflächenschicht zusätzlich Hydroxylapatit enthält.

4. Oberflächenschicht nach Anspruch 1 oder 2, **dadurch gekennzeichnet, dass** die Porendichte zwischen 10⁵ bis 10⁷ Poren/mm² liegt.

5. Oberflächenschicht nach einem der Ansprüche 1 bis 3, **dadurch gekennzeichnet, dass** das Verhältnis Ca/P über die gesamte Oberflächenschicht im Bereich von 1,0 bis 1,8 liegt.

6. Oberflächenschicht nach einem der Ansprüche 1 bis- 5, **dadurch gekennzeichnet, dass** sie einen Anteil von 30 bis 80 Atomprozenten Metalloxid enthält.

7. Oberflächenschicht nach Anspruch 6, **dadurch gekennzeichnet, dass** das Metalloxid in Form von Kristallen vorliegt, vorzugsweise mit einer Grösse der Kristalle von 10 bis 150 Nanometer.

8. Oberflächenschicht nach Anspruch 6 oder 7, **dadurch gekennzeichnet, dass** das Metaltöxid Titandioxid ist.

9. Öberflächenschicht nach Anspruch 8, **dadurch gekennzeichnet, dass** das Titandioxid in der Form von Anatas oder Rutil vorliegt.

10. Oberflächenschicht nach einem der Ansprüche 1 bis 9, **dadurch gekennzeichnet, dass** die Dicke der Schicht 0,5 bis 10,0 µm beträgt.

11. Oberflächenschicht nach einem der Ansprüche 1 bis 10, **dadurch gekennzeichnet, dass** die Poren der porösen Oberflächenschicht pharmakologisch wirksame Substanzen enthalten, vorzugsweise Peptide, Wachstumsfaktoren, Bone Morphogenetic Proteins, Antibiotika oder Entzündungshemmer.

12. Substrat mit einer Oberflächenschicht gemäss einem der Ansprüche 1 bis 11, **dadurch gekennzeichnet, dass** das Substrat eines oder mehrere der Elemente Ti, Zr, Ta, Nb, Al, V, Mg (Ventilmetalle) oder deren Legierungen enthält.

13. Substrat nach Anspruch 12, **dadurch gekennzeichnet, dass** die Oberflächenschicht mindestens teilweise aus nanokristallinen oder mikrokristallinen Oxiden oder Mischoxiden des metallischen Substrats besteht.

14. Substrat nach Anspruch 13, **dadurch gekennzeichnet, dass** der Hauptbestandteil der Oberflächenschicht aus dem nano- oder mikrokristallinen Oxid oder Mischoxid des metallischen Grundmaterials besteht, vorzugsweise mit einem Anteil von 60 bis 99 Volumen %.

15. Substrat mit einer Oberflächenschicht gemäss einem der Ansprüche 1 bis 11, **dadurch gekennzeichnet, dass** das Substrat aus Kunststoffen, vorzugsweise aus Polyoxymethylen (POM), Polyetheretherketon (PEEK), Polyaryletherketön (PAEK), Polyetherimid (PEI) oder flüssigkristallinem Polymer (LCP), Polymethylpenten (PMP), Polysulfon (PSU), Polyäthersulfon (PESU oder PES), Polyäthylenterephthalat (PETP), Polymethylmethacrylat (PMMA) oder ultrahochmolekularem Polyäthylen (UHMW-PE) besteht, wobei das Substrat mit einer metallischen Schicht aus Ventilmetallen versehen ist.

16. Verfahren zur Hersteltung einer calciumphosphathaltigen, bioaktiven, porösen Oberflächenschicht auf Ventilmetallen oder deren Legierungen wie auch Ventilmetallbeschichtungen auf einem Substrat bei dem ein zu beschichtendes Substrat anodisch einem wässrigen Elektrolyten ausgesetzt wird, in welchem Calcium- und Phosphat-lonen enthalten sind, welche in die entstehende Schicht eingelagert werden sollen und bei dem im Elektrolyten eine anodisch-plasmachemische Oberflächenmodifikation unter Funkenentladung mit Gleichspannung bzw. Gleichspannungsimpulsen und zeitlicher Änderung der Spannung erfolgt,
**dadurch gekennzeichnet, dass**
A) der wässrige Elektrolyt mit Calcium- und Phosphatzusätzen auf einen pH-Wert grösser oder gleich 9 eingestellt wird und zumindest folgende Komponenten enthält:
B1) einen oder mehrere organische Chelatbildner oder anorganische Komplexbildner im Konzentrationsbereich von 0,01 - 6,00 mol/l;
B2) eine oder mehrere Phosphatverbindungen im Konzentrationsbereich Von 0,01-6,00 mol/l, vorzugsweise 0,01 bis 0,05 mol/l;
B3) eine oder mehrere wasserlösliche Calciumverbindungen zur Einstellung des gewünschten Calcium/Phosphat-Verhältnisses im Konzentrationsbereich von 0,01 - 6,00 mol/l; und
B4) einen oder mehrere basische Zusätze im Konzentrationsbereich von 0,01 bis 6,00 mol/l zur Einstellung des gewünschten pH-Wertes.

17. Verfahren nach Anspruch 16, **dadurch gekennzeichnet, dass** der Chelatbildner eine anorganische Carbonsäure ist, vorzugsweise Bi- oder Multidentate oder deren Carboxylate.

18. Verfahren nach Anspruch 17, **dadurch gekennzeichnet, dass** die anorganische Carbonsäure aus folgender Gruppe ausgewählt ist: Zitronensäure, Weinsäure, Nitriloessigsäure (NTA), Ethylen-diamin-tetraessigsäure (EDTA), 1,2-Cyclohexandiamin-tetraessigsäure (CDTA), Diethylen-triaminessigsäure (DTPA), 2-Hydroxyethyl-ethylen-diamin-triessigsäure, Triethylentetraminhexaessigsäure (TTHA).

19. Verfahren nach Anspruch 16, **dadurch gekennzeichnet, dass** der Chelatbildner ein Keton ist, vorzugsweise ein Di- oder Multiketon.

20. Verfahren nach Anspruch 19, **dadurch gekennzeichnet, dass** das Öiketon das β-Diketon (CH₃-CO-CH₂-CO-CH₃) ist.

21. Verfahren nach Anspruch 16, **dadurch gekennzeichnet, dass** der Chelatbildner eine Organophosphorsäure oder ein Organophosphat mit vorzugsweise ≥ 2 Phosphatgruppen ist.

22. Verfahren nach Anspruch 16, **dadurch gekennzeichnet, dass** der Chelatbildner eine Organophosphonsäure oder ein Organophosphonat mit vorzugsweise ≥ 2 Phosphonatgruppen ist.

23. Verfahren nach Anspruch 16, **dadurch gekennzeichnet, dass** der Chelatbildner eine organophosphorige Säure oder ein Organophosphit mit vorzugsweise ≥ 2 Phosphitgruppen ist.

24. Verfahren nach einem der Ansprüche 16 bis 23, **dadurch gekennzeichnet, dass** der Chelatbildner als Salz vorliegt.

25. Verfahren nach Anspruch 16, **dadurch gekennzeichnet, dass** der anorganische Komplexbildner CaX₆⁴⁻ umfasst, vorzugsweise mit X = Fluorid.

26. Verfahren nach Anspruch 16 oder 25, **dadurch gekennzeichnet, dass** der Komplexbildner eine Konzentration von 0,06 bis 0,24 mol/l aufweist.

27. Verfahren nach einem der Ansprüche 16 bis 26, **dadurch gekennzeichnet, dass** die Phosphatverbindung Calcium-Bis-(Dihydrogenphosphat) ist.

28. Verfahren nach einem der Ansprüche 16 bis 27, **dadurch gekennzeichnet, dass** die Phosphatverbindung eine Konzentration 0,01 bis 0,05 mol/l aufweist.

29. Verfahren nach einem der Ansprüche 16 bis 28, **dadurch gekennzeichnet, dass** die wasserlösliche Calciumverbindung Calciumacetat ist.

30. Verfahren nach einem der Ansprüche 16 bis 29, **dadurch gekennzeichnet, dass** die wasserlösliche Calciumverbiridung eine Konzentration von 0,03 bis 0,15 mol/l aufweist.

31. Verfahren nach einem der Ansprüche 16 bis 30, **dadurch gekennzeichnet, dass** der basische Zusatz eine Hydroxidverbindung, vorzugsweise Natrium- oder Kaliumhydroxid ist.

32. Verfahren nach einem der Ansprüche 16 bis 31, **dadurch gekennzeichnet, dass** der basische Zusatz eine Konzentration von 0,5 bis 1,5 mol/l aufweist.

33. Verfahren nach einem der Ansprüche 16 bis 32, **dadurch gekennzeichnet, dass** die Parameter des Anodisierungsprozesses (Spannung, Strom, Frequenz, Beschichtungsdauer, Badgeometrie etc.) so gewählt werden, dass sich die Schicht durch Reaktion zwischen dem Substrat und dem Elektrolyten bildet, wobei die bereits gebildete Schicht durch die Funkenentladung partiell rekristallisiert.

34. Verfahren nach einem der Ansprüche 16 bis 33, **dadurch gekennzeichnet, dass** die Temperatur des Elektrolyten während des Beschichtungungsvorganges 10 bis 90 °C beträgt, vorzugsweise 20 bis 75 °C.

35. Verfahren nach einem der Ansprüche 16 bis 34, **dadurch gekennzeichnet, dass** Substrate aus den Elementen Ti, Zr, Ta, Nb, Al, V , Mg oder deren Legierungen oder auch sperrschichtbildende Metallbeschichtungen auf beliebigen Substraten beliebiger Form und Oberflächenbeschaffenheit allseitig oder partiell beschichtet werden.

36. Verfahren nach einem der Ansprüche 16 bis 35, **dadurch gekennzeichnet, dass** eine Beeinflussung der Oberflächentopographie oder -morphologie durch chemische und/oder mechanische Vorbehandlungen der Ausgangsoberfläche erfolgt.

37. Verfahren nach Anspruch 36, **dadurch gekennzeichnet, dass** die chemische Vorbehandlung ein Ätzverfahren ist.

38. Verfahren nach Anspruch 36, **dadurch gekennzeichnet, dass** die mechanische Vorbehandlung ein Strahlprozess ist.

## Claims

1. A bioactive surface layer, in particular for medical implants and prostheses, wherein
A) a variable proportion of the surface layer consists of calcium phosphate phases;
B) the surface layer contains a portion of 25 to 95 atomic percent metallic oxide of the metallic base material;
C) the thickness of the layer is between 0.1 and 50.0 µm;
D) the surface layer is porous;
E) the surface layer contains amorphous or nanocrystalline calcium phosphates; and
F) the Ca/P ratio over the entire surface layer is in the range between 0.5 and 2.0
**characterised in that**
G) the Ca-ions and PO₄⁻ions incorporated in the surface layer are distributed over the entire metal oxide layer;
H) the pore density on the surface of the surface layer is between 10⁴ and 10⁸ pores/mm²;
I) the amorphous or nanocrystalline calcium phosphates, as well as any possible hydroxyapatite portions, make up 1 to 40 volume % of the entire surface layer.

2. The surface layer according to claim 1, **characterized in that** the surface layer consists of hydroxyapatite.

3. The surface layer according to claim 1 or 2, **characterized in that** the surface layer additionally contains hydroxyapatite.

4. The surface layer according to claim 1 or 2, **characterized in that** the pore density is between 10⁵ and 10⁸ pores/mm².

5. The surface according to one of the claims 1 to 3, **characterized in that** the Ca/P ratio over the entire surface layer is in the range between 1.0 and 1.8.

6. The surface layer according to one of the claims 1 to 5, **characterized in that** it contains a proportion between 30 and 80 atomic percent metal oxide.

7. The surface layer according to claim 6, **characterized in that** the metal oxide is in the form of crystals, preferably having a crystal size of 10 to 150 nanometers.

8. The surface layer according to claim 6 or 7, **characterized in that** the metal oxide is titanium oxide.

9. The surface layer according to claim 8, **characterized in that** the titanium oxide is in the form of anatase or rutile.

10. The surface layer according to one of the claims 1 to 9, **characterized in that** the thickness of the layer is between 0.5 and 10.0 µm.

11. The surface layer according to one of the claims 1 to 10, **characterized in that** the pores of the porous surface layer contain pharmacologically active substances, preferably peptides, growth factors, bone morphogenetic proteins, antibiotics, or anti-inflammatories.

12. Substrate having a surface layer according to one of the claims 1 to 11, **characterized in that** the substrate contains one or more of the elements Ti, Zr, Ta, Nb, Al, V, Mg (valve metals) or alloys thereof.

13. Substrate according to claim 12, **characterized in that** the surface layer at least partially consists of nanocrystalline or microcrystalline oxides or mixed oxides of the metal substrate.

14. Substrate according to claim 13, **characterized in that** the main component of the surface layer consists of the nanocrystalline or microcrystalline oxide or mixed oxide of the metal substrate, preferably in a proportion of 60 to 99 volume %.

15. Substrate having a surface layer according to one of the claims 1 through 11, **characterized in that** the substrate consists of plastics, preferably polyoxymethylene (POM), polyetheretherketone (PEEK), polyaryletherketone (PAEK), polyetherimide (PEl) or liquid crystal polymer (LCP), polymethylpentene (PMP), polysulfone (PSU), polyethersulfone (PESU or PES), polyethylene terephthalate (PETP), polymethylmethacrylate (PMMA), or ultrahigh molecular weight polyethylene (UHMW-PE), where the substrate is provided with a metallic layer made from valve metals.

16. Method for fabricating a bioactive, porous, and calcium phosphate-containing surface layer on valve metals or alloys thereof, as well as valve metal coatings on a substrate, where a substrate to be coated is anodically exposed to an aqueous electrolyte containing calcium and phosphate ions, which are to be embedded into the forming layer, and where an anodic plasma-chemical surface modification takes place in the electrolyte by spark discharge using direct current voltage or direct current voltage pulses and time variation of the voltage
**characterized in that**
A) the aqueous electrolyte is brought to a pH-value larger than or equal to 9, using calcium and phosphate additives, and contains at least the following components:
B1) one or more organic chelating agents or inorganic complexing agents in a concentration range between 0.01 and 6.00 mol/L;
B2) one or more phosphate compounds in a concentration range between 0.01 and 6.00 mol/L, preferably between 0.01 and 0.05 mol/L;
B3) one or more water-soluble calcium compounds for arriving at the desired calcium/phosphate ratio of 0.01 to 6.00 mol/L; and
B4) one or more basic additives in a concentration range between 0.01 and 6.00 mol/L for arriving at the desired pH-value.

17. Method according to claim 16, **characterized in that** the chelating agent is an inorganic carboxylic acid, preferably bidentate or polydentate, or carboxylates thereof.

18. Method according to claim 17, **characterized in that** the inorganic carboxylic acid is selected from the following group: citric acid, tartaric acid, nitriloacetic acid (NTA), ethylenediaminetetraacetic acid (EDTA), 1,2-cyclohexanediaminetetraacetic acid (CDTA), diethylenetriamineacetic acid (DTPA), 2-hydroxyethylethylenediaminetriacetic acid, triethylenetetraaminehexaacetic acid (TTHA).

19. Method according to claim 16, **characterized in that** the chelating agent is a ketone, preferably a diketone or a polyketone.

20. Method according to claim 19, **characterized in that** the diketone is the β-diketone (CH₃-CO-CH₂-CO-CH₃).

21. Method according to claim 16, **characterized in that** the chelating agent is an organophosphoric acid or an organophosphate, with preferably ≥ 2 phosphate groups.

22. Method according to claim 16, **characterized in that** the chelating agent is an organophosphonic acid or an organophosphonate, with preferably ≥ 2 phosphonate groups.

23. Method according to claim 16, **characterized in that** the chelating agent is an organophosphorous acid or an organophosphite, with preferably ≥ 2 phosphite groups.

24. Method according to one of the claims 16 through 23, **characterized in that** the chelating agent is a salt.

25. Method according to claim 16, **characterized in that** the inorganic complexing agent comprises CaX₆⁴⁻, especially with X = fluoride.

26. Method according to claim 16 or 25, **characterized in that** the complexing agent has a concentration of 0.06 to 0.24 mol/L.

27. Method according to one of the claims 16 through 26, **characterized in that** the phosphate compound is calcium bis(dihydrogen phosphate).

28. Method according to one of the claims 16 through 27, **characterized in that** the phosphate compound has a concentration of 0.01 to 0.05 mol/L.

29. Method according to one of the claims 16 through 28, **characterized in that** the water-soluble calcium compound is calcium acetate.

30. Method according to one of the claims 16 through 29, **characterized in that** the water-soluble calcium compound has a concentration of 0.03 to 0.15 mol/L.

31. Method according to one of the claims 16 through 30, **characterized in that** the basic additive is a hydroxide compound, preferably sodium or potassium hydroxide.

32. Method according to one of the claims 16 through 31, **characterized in that** the basi c additive has a concentration of 0.5 to 1.5 mol/L.

33. Method according to one of the claims 16 through 32, **characterized in that** the parameters of the anodizing process (voltage, current, frequency, coating time, bath geometry, etc.) are selected so that the layer is formed by reaction between the substrate and the electrolyte, where the spark discharge results in partial recrystallization of the already formed layer.

34. Method according to one of the claims 16 through 33, **characterized in that** the temperature of the electrolyte during the coating process is 10°C to 90°C, preferably 20°C to 75°C.

35. Method according to one of the claims 16 through 34, **characterized in that** the substrates made from the elements Ti, Zr, Ta, Nb, Al, V, Mg, or alloys thereof, or else barrier-forming metal coatings on any substrates of any shape and surface condition, are coated all over or partially.

36. Method according to one of the claims 16 through 35, **characterized in that** the surface topography or morphology is manipulated by chemical and/or mechanical pretreatments of the starting surface.

37. Method according to claim 36, **characterized in that** the chemical pretreatment is an etching process.

38. Method according to claim 36, **characterized in that** the chemical pretreatment is a blasting process.

## Revendications

1. Couche de surface bioactive, notamment pour implants médicaux et prothèses médicales,
A) un pourcentage variable de la couche de surface se composant de phases de phosphate de calcium,
B) la couche de surface comportant un pourcentage atomique compris entre 25 et 95 pour cent d'oxyde métallique du matériau métallique de base;
C) l'épaisseur de la couche étant comprise entre 0,1 et 50,0 µm,
D) la couche de surface étant réalisée de manière poreuse,
E) la couche de surface comportant des phosphates de calcium amorphes ou nanocristallins; et
F) le rapport Ca/P étant compris, sur l'ensemble de la couche de surface, entre 0,5 et 2,0;
**caractérisée en ce que**
G) les ions de Ca et les ions de PO₄ emmagasinés dans la couche de surface sont répartis sur l'ensemble de la couche d'oxyde métallique;
H la densité des pores à la surface de la couche de surface est comprise entre 10⁴ et 10⁸ pores/mm²; et
I) les phosphates de calcium amorphes ou nanocristallins ainsi que les fractions d'hydroxylapatite éventuellement présentes constituent un pourcentage volumique de 1 à 40 pour cent de l'ensemble de la couche de surface.

2. Couche de surface selon la revendication 1, **caractérisée en ce que** la couche de surface se compose d'hydroxylapatite.

3. Couche de surface selon la revendication 1, **caractérisée en ce que** la couche de surface contient, en outre, de l'hydroxylapatite.

4. Couche de surface selon la revendication 1 ou 2, **caractérisée en ce que** la densité des pores est comprise entre 10⁵ et 10⁷ pores/mm².

5. Couche de surface selon l'une des revendications 1 à 3, **caractérisée en ce que** le rapport Ca/P est compris, sur l'ensemble de la couche de surface, entre 1,0 et 1,8.

6. Couche de surface selon l'une des revendications 1 à 5, **caractérisée en ce qu'**elle contient un rapport atomique de 30 à 80 pour cent d'oxyde métallique.

7. Couche de surface selon la revendication 6, **caractérisé en ce que** l'oxyde métallique est présent sous forme de cristaux, de préférence avec une taille de cristaux comprise entre 10 et 150 nanomètres.

8. Couche de surface selon la revendication 6 ou 7, **caractérisée en ce que** l'oxyde métallique est du dioxyde de titane.

9. Couche de surface selon la revendication 8, **caractérisée en ce que** le dioxyde de titane est présent sous forme d'anatase ou de rutile.

10. Couche de surface selon l'une des revendications 1 à 9, **caractérisée en ce que** l'épaisseur de la couche est comprise entre 0,5 et 10,0 µm.

11. Couche de surface selon l'une des revendications 1 à 10, **caractérisée en ce que** les pores de la couche de surface poreuse contiennent des substances actives du point de vue pharmacologique, de préférence des peptides, des facteurs de croissance, des protéines de morphogenèse osseuse (Bone Morphogenetic Proteins), des antibiotiques ou des anti-inflammatoires.

12. Substrat comportant une couche de surface selon l'une des revendications 1 à 11, **caractérisé en ce que** le substrat contient un ou plusieurs des éléments suivants : Ti, Zr, Ta, Nb, Al, V, Mg (métaux valves) ou de leurs alliages.

13. Substrat selon la revendication 12, **caractérisé en ce que** la couche de surface se compose, au moins partiellement, d'oxydes nanocristallins ou microcristallins ou d'oxydes mixtes du substrat métallique.

14. Substrat selon la revendication 13, **caractérisé en ce que** le composant principal de la couche de surface se compose, de préférence, de l'oxyde ou l'oxyde mixte nanocristallin ou microcristallin du matériau métallique de base, de préférence avec un pourcentage volumique compris entre 60 et 99 pour cent.

15. Substrat comportant une couche de surface selon l'une des revendications 1 à 11, **caractérisé en ce que** le substrat se compose de matières plastiques, de préférence de polyoxyméthylène (POM), de polyétheréther-cétone (PEEK), de cétone de polyéther (PAEK), de polyétherimide (PEI) ou polymère de cristal liquide (LCP), de polyméthylpentène (PMP), de polysulfone (PSU), de polyéthersulfone (PESU ou PES), de téréphtalate de polyéthylène (PETP), de polyméthylméthacrylate (PMMA) ou de polyéthylène à ultra-haute densité moléculaire (UHMW-PE), le substrat étant pourvu d'une couche métallique en métaux valves.

16. Procédé permettant de fabriquer une couche de surface bioactive poreuse, contenant du phosphate de calcium, réalisée sur des métaux valves ou sur leurs alliages ainsi que des revêtements en métaux valves sur un substrat, procédé lors duquel un substrat à revêtir est exposé, par anodisation, à un électrolyte aqueux qui contient des ions de calcium et des ions de phosphate qui doivent être emmagasinés dans la couche en genèse, et lors duquel a lieu, dans l'électrolyte, une modification de surface anodique-plasmachimique sous décharge disruptive avec tension continue ou avec impulsions de tension continue et changement de l'intensité de la tension dans le temps,
**caractérisé en ce que**
A) l'électrolyte aqueux est ajusté à une valeur de pH supérieure ou égale à 9 grâce à l'ajout de calcium et de phosphate et contient au moins les composants suivants :
B1) un ou plusieurs agents chélateurs organiques ou agents complexants inorganiques avec une concentration comprise entre 0,01 et 6,00 mol/l;
B2) un ou plusieurs composés de phosphate avec une concentration comprise entre 0,01 et 6,00 mol/l, de préférence entre 0,01 et 0,05 mol/l;
B3) un ou plusieurs composés de calcium hydrosolubles permettant d'ajuster le rapport calcium/phosphate souhaité sur une concentration comprise entre 0,01 et 6,00 mol/l; et
B4) un ou plusieurs additifs basiques avec une concentration comprise entre 0,01 et 6,00 mol/l afin d'ajuster la valeur de pH souhaitée.

17. Procédé selon la revendication 16, **caractérisé en ce que** l'agent chélateur est un acide carboxylique inorganique, de préférence un acide bidentate ou multidentate, ou leurs carboxylates.

18. Procédé selon la revendication 17, **caractérisé en ce que** l'acide carboxylique anorganique est sélectionné parmi les groupes suivants : de l'acide citrique, de l'acide tartrique, nitriloacétate (NTA), de l'acide éthylènediaminetétraacétique (EDTA), de l'acide cyclohexanediaminetétraacétique-1,2 (CDTA), de l'acide diéthylènetriamineacétique (DTPA), de l'acide hydroxyéthyléthylènediaminetriacétique-2, de l'acide triéthylènetetraminehexacétique (TTHA).

19. Procédé selon la revendication 16, **caractérisé en ce que** l'agent chélateur est une cétone, de préférence une dicétone ou une multicétone.

20. Procédé selon la revendication 19, **caractérisé en ce que** la dicétone est la dicétone β (CH₃-CO-CH₂-CO-CH₃).

21. Procédé selon la revendication 16, **caractérisé en ce que** l'agent chélateur est un acide organophosphorique ou un organophosphoré avec, de préférence, ≥ 2 groupes de phosphates.

22. Procédé selon la revendication 16, **caractérisé en ce que** l'agent chélateur est un acide organophosphonique ou un organophosphonate avec, de préférence, ≥ 2 groupes de phosphonates.

23. Procédé selon la revendication 16, **caractérisé en ce que** l'agent chélateur est un acide organophosphoreux ou un organophosphites avec, de préférence, ≥ 2 groupes de phosphites.

24. Procédé selon l'une des revendications 16 à 23, **caractérisé en ce que** l'agent chélateur se présente sous forme de sel.

25. Procédé selon la revendication 16, **caractérisé en ce que** l'agent complexant inorganique comprend du CaX₆⁴⁻, de préférence avec X = fluorure.

26. Procédé selon la revendication 16 ou 25, **caractérisé en ce que** l'agent complexant présente une concentration comprise entre 0,06 et 0,24 mol/l.

27. Procédé selon l'une des revendications 16 à 26, **caractérisé en ce que** le composé de phosphate est du bis-(dihydrogène-phosphate) de calcium.

28. Procédé selon l'une des revendications 16 à 27, **caractérisé en ce que** le composé de phosphate présente une concentration comprise entre 0,01 et 0,05 mol/l.

29. Procédé selon l'une des revendications 16 à 28, **caractérisé en ce que** le composé de calcium hydrosoluble est le l'acétate de calcium.

30. Procédé selon l'une des revendications 16 à 29, **caractérisé en ce que** le composé de calcium hydrosoluble présente une concentration comprise entre 0,03 et 0,15 mol/l.

31. Procédé selon l'une des revendications 16 à 30, **caractérisé en ce que** l'additif basique est un composé d'hydroxyde, de préférence de l'hydroxyde de sodium ou de l'hydroxyde de potassium.

32. Procédé selon l'une des revendications 16 à 31, **caractérisé en ce que** l'additif basique présente une concentration comprise entre 0,5 et 1,5 mol/l.

33. Procédé selon l'une des revendications 16 à 32, **caractérisé en ce que** les paramètres du processus d'anodisation (tension, courant, fréquence, durée du processus de revêtement, géométrie du bain etc.) sont sélectionnés de telle sorte que la couche se forme par réaction entre le substrat et l'électrolyte, la couche déjà formée recristallisant partiellement sous la décharge disruptive.

34. Procédé selon l'une des revendications 16 à 33, **caractérisé en ce que**, pendant le processus de revêtement, la température de l'électrolyte est comprise entre 10 et 90 °C, de préférence entre 20 et 75 °C.

35. Procédé selon l'une des revendications 16 à 34, **caractérisé en ce que** des revêtements sont réalisés, soit sur toutes les faces, soit de manière partielle, sur des substrats composés des éléments suivants : Ti, Zr, Ta, Nb, Al, V, Mg ou de leur alliages ou bien encore sur des revêtements métalliques formant des couches d'arrêt par rapport à n'importe quels substrats de n'importe quelle forme et nature de surface.

36. Procédé selon l'une des revendications 16 à 35, **caractérisé en ce que** la topographie ou morphologie de surface est influencée par des prétraitements chimiques et/ou mécaniques de la surface d'origine.

37. Procédé selon la revendication 36, **caractérisé en ce que** le prétraitement chimique est un procédé de corrosion.

38. Procédé selon la revendication 36, **caractérisé en ce que** le prétraitement mécanique est un procédé de grenaillage.
